# EUROPEAN PATENT SPECIFICATION

(11) **EP 2 114 432 B1**
(45) Date of publication and mention of the grant of the patent: **30.07.2014**
(21) Application number: 07854968.0
(22) Date of filing: 05.12.2007
(51) Int. Cl.: A61K 38/17, A61K 31/196, A61K 39/395, A61P 5/00, A61K 45/06

(54) **METHODS OF INCREASING CANCER SENSITIVITY TO CHEMOTHERAPEUTIC AGENTS USING CHIMERIC ISF35**
VERFAHREN ZUR ERHÖHUNG DER EMPFINDLICHKEIT VON KREBS AUF CHEMOTHERAPEUTISCHE MITTEL MIT CHIMÄREM ISF35
PROCÉDÉS POUR RENDRE LES CANCERS PLUS SENSIBLES AUX AGENTS CHIMIOTHÉRAPEUTIQUES UTILISANT L'ISF35 CHIMÉRIQUE

(30) Priority: 05.12.2006 US 868716 P
(43) Date of publication of application: 11.11.2009
(73) Proprietor: Memgen LLC, San Diego, CA 92101 (US)
(72) Inventor: PRUSSAK, Charles E., San Diego, CA 92037 (US)
(74) Representative: Vossius & Partner
(86) International application number: PCT/US2007/086550
(87) International publication number: WO 2008/070743

(56) References cited:
- WO-A1-03/099340
- US-A1- 2002 039 557
- US-A1- 2003 220 473
- DATABASE BIOSIS [Online] BIOSCIENCES INFORMATION SERVICE, PHILADELPHIA, PA, US; November 2006 (2006-11), WIERDA WILLIAM G ET AL: "Membrane-stable, humanized CD154 gene therapy for patients with CLL.", XP002614458, Database accession no. PREV200700258855
- DICKER F. ET AL.: "CD154 induces p73 to overcome the resistance to apoptosis of chonic lymphocytic leukemia cells lacking functional p53.", BLOOD, vol. 108, no. 10, 15 November 2006 (2006-11-15), pages 3450-3457, XP002614459,

## Description

### FIELD OF THE INVENTION

This invention relates to the field of cancer and its treatment. In particular, the invention relates to treatment of cancer by administration of nucleic acids encoding a chimeric protein.

### BACKGROUND OF THE INVENTION

An immune reaction typically begins with a T lymphocyte (T cell) that has on its surface a T cell receptor (TCR) that binds to an antigen derived peptide associated with a class 11 major histo-compatibility complex (MHC) molecule. The T cell also expresses on its surface various polypeptides, which are referred to as "ligands" because they bind to receptors on cells associated with an immune-mediated response, as described in more detail below. When the T cell receptor binds to a MHC-associated antigen, such as antigen from a malignant cell, it becomes activated and expresses a ligand on its surface. The ligand is only present on the cell surface for a short time, and once it has been removed front the surface of the cell, the T cell ability to bind a receptor-bearing cell is lost. One such ligand is called CD154.

CD154 is one member of a larger family of ligands, collectively referred to as the TNF superfamily (Gruss et al, Cytokines Mol Ther, 1:75-105, 1995 and Locksley et al, Cell, 104:487-501, 2001). Members of the TNF superfamily include Fas ligand ("FasL"), TNFα, LTα, lymphomicin (TNFβ), CD154, TRAIL, CD70, CD30 ligand, 4-tBB ligand, APRIL, TWEAK, RANK ligand, LIGHT, AITR ligand, eclodysplasin, BLYS, VEG1, and OX40 ligand TNF superfamily members share a conserved secondary structure comprising four domains: domain. I, the intracellular domain; domain II, which spans the cell membrane and is known as the transmembrane domain; domain III, which consists of the extracellular amino acids closest to the cell membrane; and domain IV, the distal extracellular domain (Kipps et al., WO98/26061 published Jun. 18, 1998). Typically, at least a part of domain IV can be cleaved from the parent molecule. The cleaved fragment often exhibits the same biological activity of the intact ligand and is conventionally referred to as a "soluble form" of the TNF family member:

### SUMMARY OF THE INVENTION

The invention relates to the embodiments as characterized in the claims. The present invention relates to the use of chimeric CD154 polypeptides and nucleic acids encoding them for cancer treatment. The chimeric CD154 can be capable of expression on diverse cell types, including B cells. In some embodiments, the chimeric CD154 is less resistant to proteolytic cleavage and thus more stable when expressed on cellular membranes. Additionally, chimeric CD154 can maintain the receptor-binding capabilities of human CD154; and thus elicit a similar type of immunological response in humans.

Chimeric TNF or CD154 molecule made of portions derived from human molecules combined with molecules from nonhuman origin are contemplated for use with the invention. The useful chimeric molecules may contain multiple segments derived from human or nonhuman sources.

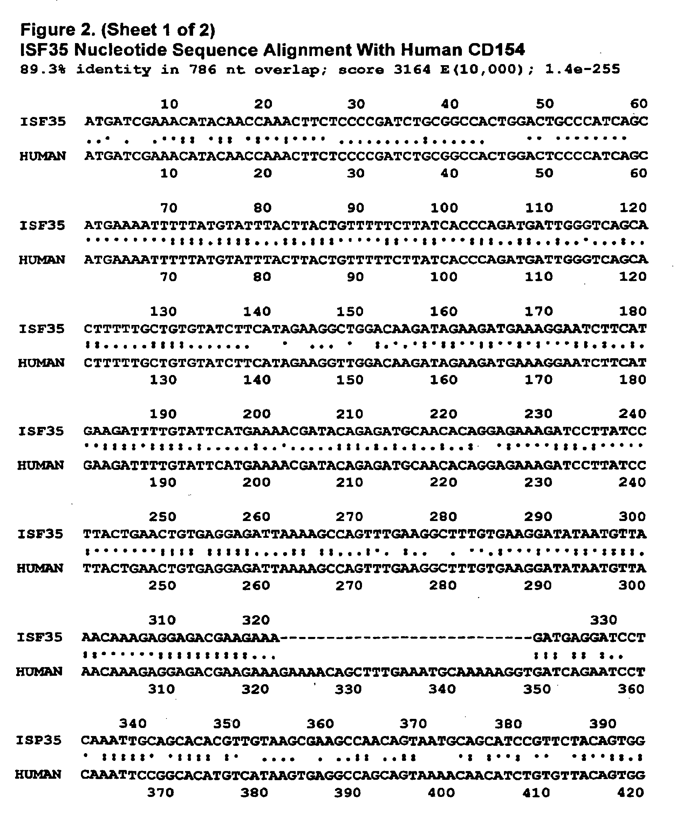

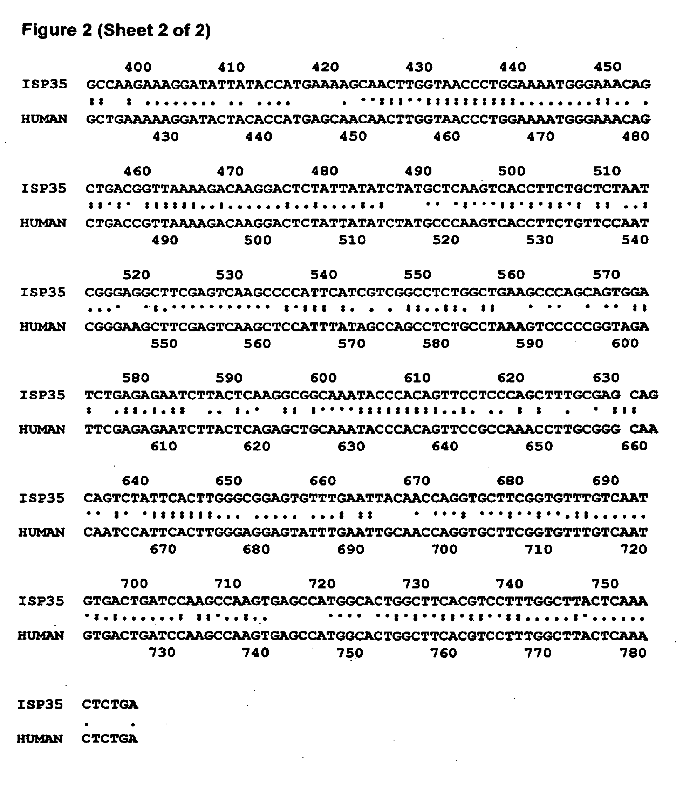

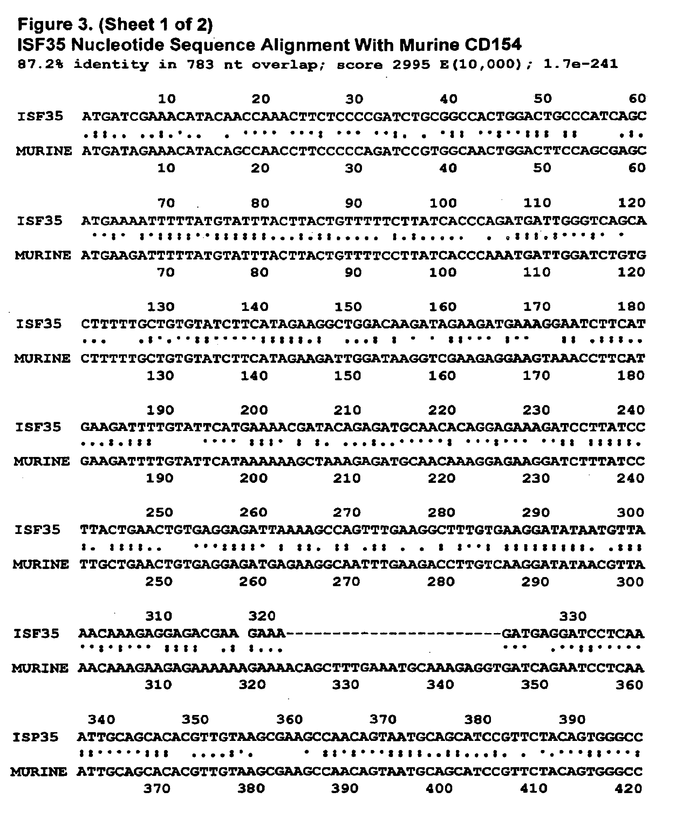

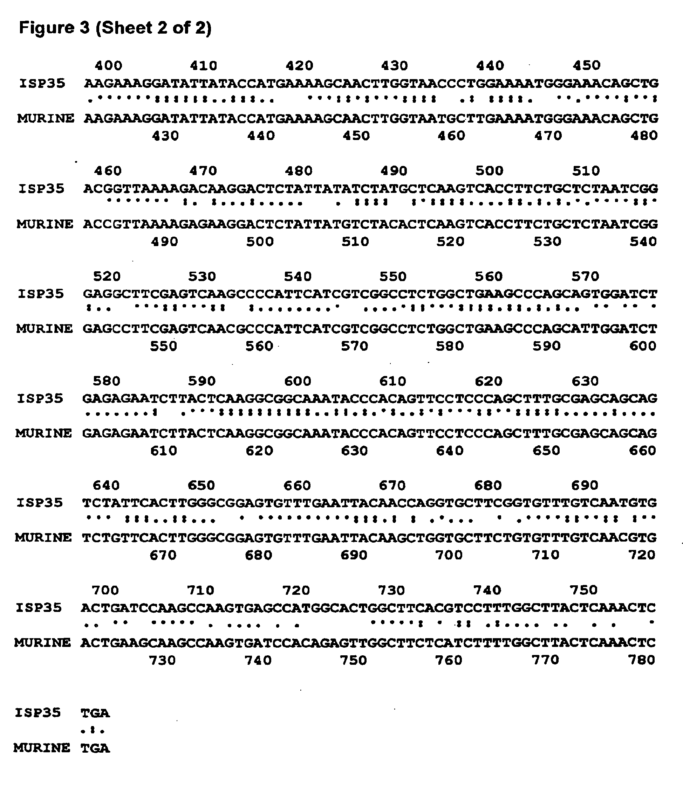

More specifically, in some embodiments of the invention, and methods of the invention, relates to, or the use of, the chimeric CD154 sequence ISF35, which is a mouse/human chimeric sequence shown in SEQ ID NO: 1, and in Figures 2 and 3.

In some embodiments, a means of treating cancer in a patient is provided by administering to the patient an effective amount of ISF 35, and analogous constructs, prior to administering to the patient an effective amount of a chemotherapeutic agent thereby making the cancer more sensitive to the chemotherapeutic agent than prior to administering ISF 35, or an analogous construct, to the patient as defined in the claims. The ISF35 or an analogous construct can be administered prior to administration of the chemotherapeutic agent. The JSF35 or an analogous construct can be administered before the chemotherapeutic agent. An "analogous construct" to ISF35 as used herein refers to another chimeric CD154 molecule according to the present invention. Further, and additionally, an analogous construct includes constructs having a substantial homology to ISF35. In some embodiments, this includes having at least a 98% homology with ISF35. In other embodiments, this includes having at least an 70%, 75%, 80%, 85%, 90%, 95%, 96%, 97% or 98% or more homology with ISF35.

In further embodiments, a means of treating a refractory cancer in a patient is provided by administering to the patient an effective amount of ISF35 or an analogous construct prior to administering to the patient an effective amount of a chemotherapeutic agent thereby making the refractory cancer more sensitive to the chemotherapeutic agent than prior to administering ISF 35 or an analogous construct to the patient as defined in the claims. The ISF35 or an analogous construct can be administered prior to administration of the chemotherapeutic agent. The ISF35 or an analogous construct can be administered before the chemotherapeutic agent.

In some embodiments, a means of increasing the sensitivity of a cancer in a patient to chemotherapy is provided by administering ISF35 or an analogous construct to the patient prior to administering chemotherapy.

Also described herein is a method of increasing sensitivity to radiation treatment, for example for treatment of cancer, is provided by administering to a patient an effective amount of ISF 35 or an analogous construct prior to administering to the patient a radiation treatment.

In additional embodiments, the ISF 35 or an analogous construct can be administered at a dosage of from about 1X10⁵ to about 1X10¹² viral particles with certain of embodiments of the invention described herein.

The cancer for treatment using the present invention can be selected, for example, from breast cancer, lung cancer, colon cancer, prostate cancer, skin cancer, colon cancer, prostate cancer, skin cancer, urinary bladder cancer, lymphoma, oral cavity cancer, pharynx cancerr; leukemia, kidney cancer, pancreatic, cancer, esophageal cancer, rectal cancer, bronchial cancer, and stomach cancer.

The chemotherapeutic agent can be selected from, for example, the group consisting of fludarabine, alemtuzimab, chlorumbucil, and rituximab.

In some embodiments, radiation is also administered to the patient.

In further embodiments, a means of treating cancer in a patient by administering to the patient an effective amount of a chimeric CD154 polypeptide or nucleic acid encoding it prior to administering to the patient an effective amount of a chemotherapeutic agent thereby making the cancer more sensitive to the chemotherapeutic agent than prior to administering chimeric CD154 polypeptide to the patient as defined in the claims.

In yet further embodiments, a means of treating cancer in a patient is provided, by administering a chimeric CD154, polypeptide or nucleic acid encoding it prior to administering to said patient an effective amount of a chemotherapeutic agent thereby making the cancer more sensitive to the chemotherapeutic agent than prior to administering chimeric CD154 polypeptide to the patient.

Also described is a method of treating cancer in a patient is provided by administering to the patient an effective amount on a molecule that binds to the GD40 protein.

Also described is a method of treating cancer in a patient is provided by administering to the patient an effective amount of an antibody that binds to the CD40 protein.

### BRIEF DESCRIPTION OF THE DRAWINGS

FIGS. 1a and 1b are schematic diagrams that show polynucleotides encoding various chimeric CD154 sequences, and indicates the location of subdomains associated with specific properties of the chimeric CD154. The domains or subdomains derived from murine CD154 are shown shaded. The ISF 35 chimera is shown in FIG 1b.
Fig. 2 is the nucleotide sequence of ISF35 [SEQ ID NO. 1] aligned against human CD154. Regions homologous with human CD154 are indicated by bold type.
Fig. 3 shows the nucleotide sequence of ISF35 [SEQ ID NO. 1] aligned against murine CD154. The ISF35 nucleotide, sequence is the upper sequence in the alignment, while the nucleotide sequence for the human or mouse CD154 is the lower sequence.

### DETAILED DESCRIPTION OF THE INVENTION

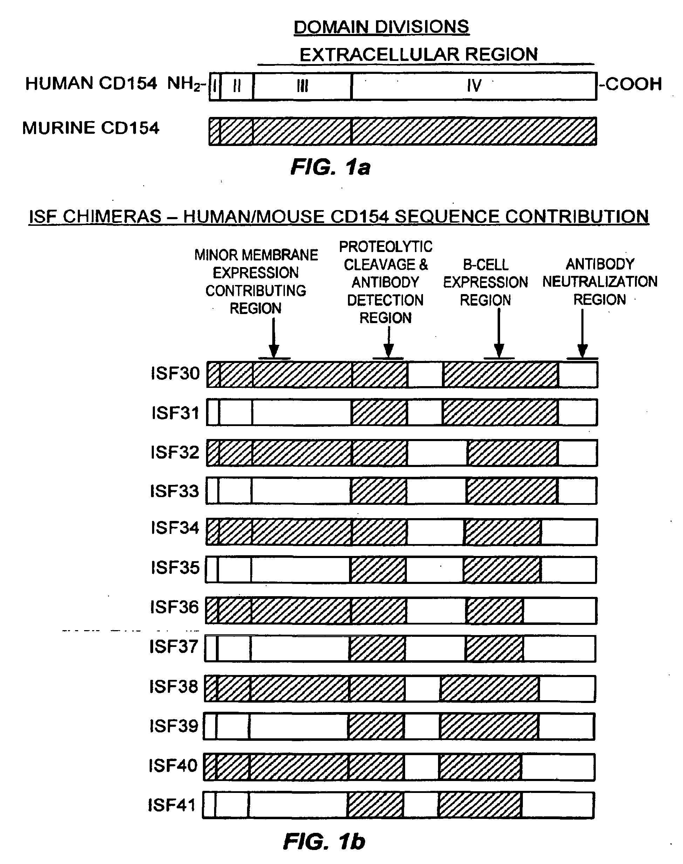

The present invention features a means of treating cancer in a patient by treating the patient with a nucleic acid encoding a chimeric CD154 protein. A preferred nucleic acid is ISF 35. which is a chimeric mouse/human sequence shown in FIG 1 in a representative manner, and more specifically in FIGS. 2 and 3 (SEQ ID NO: 1). A comparison between ISF35 and the human CD154 sequence is shown in Figure 2, while a comparison-between ISF35 and the murine CD154 sequence is shown in Figures 1 and 2. The ISF 35 is to be administered in combination with a chemotherapeutic agent. ISF 35 is to be administered prior to administration of the chemotherapeutic: agent.

The features of the invention are set forth with particularity herein and in the appended claims. A better understanding of the features and advantages of the present disclosure will be obtained by reference to die following detailed description that sets forth illustrative embodiments, in which the principles described herein are utilized.

### Definitions

A disclosed composition is to be administered to a patient in a therapeutically effective amount. As used herein, the terms "therapeutically effective amount" and "therapeutically effective dose" refer to the amount of an active agent, for example, ISF35, required to be administered in order to induce a desired result in the patient That result can be, for example, alleviation or amelioration (complete or partial) of the symptoms of the condition of the patient in need of treatment, any other desired improvement in the patient's symptoms, disease or condition, or prophylaxis or delay in the onset of symptoms of cancer.

As used herein, the term "corresponding" refers to the sequence of nucleotides or amino acids of CD154 of one species that is homologous to is nucleotide or amino acid sequence of CD154 of another species. This homology is based on the similarity in secondary structure, such as the location of domain boundaries, among CD154 of different species.

As used herein, the term "expression vector" refers to a nucleic acid that expresses, or directs the expression of, a recombinant nucleotide sequence and that is capable of infecting cells and replicating itself therein. Typical expression vectors include plasimds used in recombinant DNA technology and various viruses capable of replicating within bacterial or animal cells. A number of expression vectors have been described in the literature. Cantwell et al., Blood. In (1996) entitled "Adenovirus Vector Infection of Chronic Lymphocytic Leukemia B Cells;" Woll, P. J. and I. R. Hart, Ann. Oncol., 6 Suppl 1.73 (1995); Smith, K. T., A. J. Shepherd, J. E. Boyd, and G. M. Lees, Gene Ther., 3:190 (1996); Cooper. M. J., Semin Oncol., 23:172(1996); Shaughnessy E., D. Lu, S. Chalterjee, and, K. K. Wong, Semin Oncol., 23:159 (1996), Glorioso, J. C., N.A, DeLuca, and D. J. Fink, Annu. Rev. Microbiol., 49:675 (1995); Flotte, T. R. and B.J. Carter, Gene Ther., 2:357

(1995); Randrianarison-Jewtoukoff, V. and M. Perricaudet, Biologicals., 23:145 (1995); Kohn, D. B., Curr. Opin. Pediatr., 7:56(1995); Vile, R. G. and S. J.'Russell, Br. Med. Bull., 51:12 (1995); Russell, S. J., Semin. Cancer Biol., 5:437 (1994); and Ali, M., N. R. Lemoine, and C. J. Ring, Gene Ther., 1:367 (1994).

The term "method of treating" or similar phrases when used in relation to the present invention means amelioration, prevention or relief from the symptoms and/or effects associated with a condition in an individual.

As used herein, the term "therapeutically effective amount" can also refer to the quantity or active agent or therapeutically effective substance, such as ISF35, which results in improvement of the patient's symptoms, disease, or condition, where tittle or no improvement would occur in the absence of the active agent. Typically, the therapeutically effective substance is administered for a sufficient period of time to achieve the desired therapeutic effect. The term "therapeutically effective amount" can also refer to the quantity of active agent or therapeutically effective substance, such as ISF35, which results in improvement or stasis of the patient's symptoms, disease, or condition, where less, little or no improvement would occur in the absence of the active agent.

Therapeutic efficacy and the therapeutically effective dose of ISF35 or an analogous construct can be determined by using standard pharmacological procedures. Such procedure can be performed in experimental animals to determine such doses.

The dosage regimen of ISF35 or an analogous construct can be selected in accordance with a variety of factors including type, species, age, weight, sex, medical condition of the patient, the severity of the condition, the route of administration, and the particular composition or formulation employed.

Generally, a therapeutic amount of ISF35 or an analogous construct, or a recombinant construct thereof comprising the sequence of ISF35 or an analogous construct, is in the range of from about 1X10⁷ to about 1X10¹² viral particles. The concentration of the viral preparation can be from about 5X10³ to about 5X10¹² viral particles per ml. This dosage regimen can be adjusted to provide the optimal therapeutic response.

The compositions can be administered either alone or in combination with one or more additional agents.

### CLL

Chronic Lymphocytic Leukemia (CLL) is a disease that results in the accumulation of clonal, mature B-lymphocytes in peripheral blood and lymph nodes. This disease was initially thought to be primarily a disease of the elderly and was characterized as having an indolent course. As a result, early therapeutic intervention was thought not to be necessary and the patients were told that they would probably die from causes unrelated to CLL before treatment was necessary.

Recent developments in clinical detection and improved paradigms for the diagnosis of CLL have shown that the early concepts regarding the benign nature of this disease were incorrect. It is now known that CLL patients are particularly susceptible to infectious diseases, autoimmune disorders and increased risk for secondary malignancies. In contrast to the earlier prognosis of this disease, 50% of the CLL patients with early-diagnosed disease will progress at an expedited rate and the survival period for these patients measured in months.

Because of the pernicious nature of this disease, a number of treatment paradigms have been developed. Even though there is no cure for this disease, CLL patients are usually first treated with the alkylating agents chlorambucil and cyclophosphamide or the purine analogues fludaribine, pentostatin and cladrabine as single agents or in combination. Fludaribine is the principal first line treatment for CLL with an overall response rate approaching 70%. Even though the CLL patients' overall response rate (2-10%) to the alkylating agents is lower when compared to Fludaribine, there is no apparent survival benefit from patients treated with either regimen.

In an attempt to improve survival outcomes, new CLL directed protocols have been developed using a "cocktail" approach. In this manner the chemotherapies (fludarabine or pentostalin and cyclophosphamide) are combined with a CD19 targeted chemoimmunotherapy (rituximab) resulting-in response and complete response rates approaching 90% and 60% respectively. Even with the elevated response rates to these treatment protocols, in many patients the disease will recur necessitating further treatments. The need for subsequent treatments eventually leads to resistance to these agents. In these cases, another chemoimmunotherapy alemtuzumab (Campath®) that targets CD52 on the malignant B cells can be used for salvage therapy.

In addition to CLL there are other therapies used to treat B-cell-mediated leukemias and lymphomas. These include many of the chemotherapies used to treat solid tumor cancers used as single agents or in combinations. The agents used for the B-cell malignancies may include cisplatin, asparaginase, dacarbazine, mechlorethamine, mephalan, cytarabine, etoposide, carmustine, procarbazine, lomustine, dactinosmycin, plicamycin, methotrexate, carboplatin, doxorubicin and daunorubicin among others.

Along with the long established chemotherapies, novel anti-leukemic agents including Imatinib mesylate (Gleevec®) and dasatinib are particularly effective in patients with Chronic Lymphocytic-Leukemia (CLL). In addition to these agents many monoclonal antibodies are being developed to target the Leukemia associated antigens CD22, CD40 or C200 among others.

### CD154

An immune reaction typically begins with a T lymphocyte (T cell) that has on its surface a T cell receptor (TCR) that binds to an antigen derived peptide associated with a class II major histo-compatibility complex (MHC) molecule. The T cell also expresses on its surface various polypeptide, which are referred to as "ligands" because they bind to receptors on cells associated with an immune-mediated response, as described in more detail below. When the T cell receptor binds to a MHC associated antigen, such is antigen derived from a malignant cell, it becomes activated and expresses a ligand on its surface. The ligand is only present on the cell surface for a short time, and once it has been removed from the surface of the cell, the T cell's ability to bind a receptor-bearing cell is lost. One such ligand is called CD154.

CD154 is one member of a larger family af ligands, collectively referred to as the TNF superfamily (Cruss et al, Cytokines Mol Ther, 1:75-105, 1995 and Locksley et al, Cell, 104:487-501, 2001). Members of the TNF superfamily include Fas ligand ("FasL"), TNF.alpha., LT alpha., lymphotoxin (TNF beta), CD154, TRAIL, CD70, CD30 ligand, 4-1BB ligand, APRIL, TWEAK, RANK ligand, LIGHT, AITR ligand, cetodysplasin, BLYS, VEGI, and OX40 ligand. TNF superfamily members share a conserved secondary structure comprising four domains: domain 1, the intracellular domain; domain II, which spans the cell membrane and is known as the transmerabrane domain; domain III, which consists of the extracellular amino acids closest to the cell membrane; and domain IV, the distal extracellular domain (Kipps et al., WO98/26061 published Jun. 18, 1998). Typically, at least a part of domain IV can be cleaved from the parent molecule. The cleaved fragment often exhibits the same biological activity of the intact ligand and is conventionally referred to as a "soluble form" of the TNF family member.

### Biological Activity of CD154

The interactions between CD154 (also known as CD40 ligand) and its cognate receptor, CD40, are critical for immune recognition. (Banchereau J. et al., Annu. Rev. Immunol. 12:881-922, 1994; Laman J. D. et al., Crit. Rev. Immunol., 16:59-108, 1996). CD154 is transiently expressed on CD4.sup + T cells following T cell receptor engagement by antigen presenting cells through MHC class II molecules. (Roy M. et al., J. Immunol., 151:2497-2510, 1993; Hepmann P. et al., Eur. J. Immunol., 23:961-964, 1993; Castle B. E. et al., J. Immunol., 151:1777-1788, 1993; Contwell M. et al., Nat. Med., 3:984-989, 1997). This, in turn. can cause activation of CD40-expressing antigen presenting cells (APCs), including B cells, dendritic-cells, monocytes. and macrophages. (Ranheim E. A. et al., J. Exp. Med., 177:925-935, 1993; Ranheim E. A. et al., Cell. Immunol., 161:226-235, 1995). Such CD40 activated cells can set off a cascade of immune activating events that lead to a specific and effective immune response against foreign antigens, such as viruses or tumors. The importance of interactions between CD40 and CD154 is underscored by the finding that individuals who have inherited defects in the ligand for CD40 have profound immune deficiency (Korthauer J. et al., Nature, 361:539-541, 1993, Aruffo A. et al., Cell., 72:291-300, 1993). Such patients have an immune deficiency syndrome associated with impaired germinal center formation, defective isotype switching, and marked susceptibility to various bacterial and viral pathogens.

Because CD154 is such critical molecule in immune regulation, several mechanisms control human CD154 expression. First, membrane-expressed CD154 can be cleaved and an extracellular portion of CD154 capable of binding the CD154 receptor, CD40, is released as a soluble molecule. Proteolytic cleavage enzymes have been shown to cleave human CD154 at different sites along the ligand, and release a soluble form of CD154 that is capable of binding to CD40 and stimulating an immune response. (Pietravalle F. et al., J. Biol. Chem., 271:5965-5967, 1996; Pietravalle F. et al, Eur. J. Immunol., 26:725-728, 1996). For instance, one study has shown that CD 154 is cleaved between Phe 111 and Ala 123 (Pietravalle F. et al., Eur. J. Immunol., 26:725-728, 1996), and cleavage has also been reported at Met 113. Second, CD154 interaction with its cognate receptor can induce rapid downmodulation of CD154 surface expression. (Cantwell M.-et al., Nat. Med., 3:984-989, 1997). Third, CD154 gene transcription is tightly regulated with maximum ligand expression 4 to 6 hours after TCR ligation followed by rapid decreases in CD154 RNA and protein synthesis. (Id.) Together, these regulatory mechanisms ensure specificity of an immune response to a specific antigen. The importance of maintaining tight control of CD 154 expression is illustrated in individuals with systemic lupus erythematosus (SLE). These patients appear to hyper-express CD154 as well as possess elevated levels of soluble CD154 in their plasma, suggesting uncontrolled CD154 expression contributes to SLE disease activity. (Kato K, et al., J. Clin. Invest., 101:1133-1141, 1998; Vakkalanka R. K., Arthritis Rheum., 42:871-881, 1999).

Studies manipulating B cells and other tumors work by either-enhancing the antigen presentation of the neoplastic cell itself, as is the case for CLL and B cell lymphoma, or by activating bystander antigen presenting cells, such as dendritic cells that can initiate an anti-tumor immune response, as is the case for CD40-negative tumors. However, additional studies also suggest CD154 might have a direct growth-inhibitory effect on certain tumors, especially carcinomas of the breast. (Tong A. W. et al., Clin. Cancer Des., 7:691-703, 2001; Hirano A., Blood, 93:2999-3007, 1999). In addition, there is evidence that growth of some types of lymphoma can be directly inhibited by CD40; ligation. (Wilsey J. A. et al., J. Immunol., 158:2932-2938, 1997). As such, a wide range of tumors should be amenable to CD154 immunotherapy.

Given the similarities in nucleotide sequences coding for CD154 molecules of different species, such as human, mouse and cow, a nucleotide sequence encoding one domain or subdomain of CD154 from one species is interchangeable with the corresponding nucleotide sequence of CD154 front another species to result in a hybrid polynucleotide sequence that encodes a chimeric CD154. One such chimeric molecule is ISF35, a mouse/human CD154 chimera which is shown in a representative manner in FIG. 1, and the specific sequence in FIGS. 2 and 3 [SEQ. ID NO. 1].

In some embodiments, the nucleotide sequences that are exchanged for corresponding sequences between species are selected for functional reasons, i.e., because the selected sequence encodes a domain or subdomain that cither provides or modifies a desired function, or eliminates an undesired function of the target ligand gene.

In the art it is believed that at least part of human CD154 is cleaved from the parent molecule and becomes a soluble molecule. In the context of this invention, the soluble form is generally undesirable. Thus, in some embodiments of the invention, exchanging an amino acid, or amino acid sequence, of human CD154 that comprises a cleavage site recognized by proteolytic enzymes with an amino acid, or amino acid sequences. of non-human CD154, that does not contain this cleavage site, can at least partially ameliorate that problem. Preferably, the non-human CD154 is murine CD154.

According to some embodiments of the invention, an extracellular domain of human CD154 includes at least one amino acid, or a sequence of amino acids, at or near the border of domain III and domain IV that is recognized and cleaved by cleavage proteases. According to some embodiments of the present invention, at least one such cleavage site exists between nuelcotides 322-348, amino acids 108-116, of human CD154.

Moreover, according to some embodiments of the invention, an extracellular domain of human CD154 can include at least one amino acid, or a sequence of amino acids, that binds to a human CD154 receptor, e.g., CD40. For this reason, in some embodiments, even the soluble form of CD154 is capable of binding CD154 receptors on antigen presenting cells and may activity participate in an immune response. Thus, this extracellular region of human CD154 in certain embodiments are preferably conserved in order to maintain native CD 154 receptor binding.

Accordingly, a presently preferred embodiment of the present invention is a chimeric CD154 polynucleotide sequence comprising a first nucleotide sequence encoding an extracellular subdomain of non-human CD154 that corresponds to and replaced a cleavage site of human CD154. According to some embodiments of the invention, replacing a a subdomain of human CD154 containing a CD154 cleavage site with the corresponding subdomain of non-human CD154 results in a chimeric CD154 that is less, and in some embodiments, substantially less, susceptible to cleavage than human CD154.

In some embodiments of invention, the first nucleotide sequence can be operatively linked to a second nucleotide sequence that encodes an extracellular sub-domain of human CD154 involved in binding to a human CD154 receptor, such as the CD40 ligand. In this way, the polynucleotide sequence of some embodiments provided by the present invention can encode a chimeric CD154 that binds to human cells expressing the CD154 receptor.

Moreover, according to some embodiments of the invention, an extracellular domain of murine and human CD154 can include at least one amino acid, or a sequence of amino acids, that allows expression of the molecule on the membranes of murine and human cells. For instance, both murine and human CD154 are expressed by HeLa cells. However, murine CD154 is expressed by a greater variety of cells, including human cells, as compared to human CD154. In fact murine CD154 may be expressed in human cells that typically do not express human CD154, such as human CD40+ cells, particularly CLL cells. This differential expression between human and marine CD154 has also been described. Thus, in some embodiments of the invention, exchanging an amino acid, or sequence thereof, of human CD154 that is involved in expression of the human molecule with an amino acid, or sequence thereof, of murine CD154 involved in expression of the non-human molecule can at least partially address this problem.

Accordingly, in a preferred embodiment of the present invention, a chimeric CD154 polynucleotide sequence comprises a first nucleotide sequence that further encodes an extracellular subdomain of murine CD154 that is critical for expression of the murine CD154 molecule by murine and human cells. In this way, the polynucleotide sequence provided by some embodiments of the present invention can encode a chimeric CD154 that is capable of expression by a variety of cell types, including human CD40+ cells that do not typically express human CD154. Although this embodiment can, and preferably does, involve the use of murine CD154, in some embodiments of the present invention the use of other non-human CD154 that may be expressed by human cells can be used.

Further, according to some embodiments of the invention, an extracellular domain of murine CD154 can include an amino acid, or a sequence of amino acids, involved in detecting the expression of the chimeric CD154 because it binds to murine CD154 specific antibody. In this way, the expression of the chimeric CD154 polynucleotide sequence can be specifically detected, typically by FACS or immunohistochemistry, and thereby be distinguished from expression of native human CD154.

Accordingly, in a preferred embodiment of the present invention, the chimeric CD154 polynucleotide sequence comprises a first nucleotide sequence that further encodes an extracellular subdomain of non-human CD 154 that detects the expression of chimeric CD154 by binding to anti-murine CD154 antibodies.

In a preferred embodiment of the present invention, this first nucleotide sequence can encode a subdomain of domain IV of non-human CD154, preferably murine, although other sequences may be used alone or in conjunction. This subdomain IV of murine CD154 comprises the amino acid sequences that replace the cleavage site of human CD154, that are thought to be critical for expression of the murine molecule by murine and human cells, and that are involved in detection of the chimeric CD 154 in some embodiments of of the present invention. In addition, and in some embodiments of the invention, this first nucleotide sequence may encode a subdomain of domain III of non-human CD154 that is at or immediately adjacent to the border of domains III and domain IV. According to some embodiments of the present invention, this subdomain comprises a portion of a cleavage site of human CD154.

Preferably in some embodiments, the first nucleotide sequence can further encode domains I, II and III of murine CD154, because this construct is believed to result in improved expression of the chimeric CD154 by human cells. In some embodiments of the invention, the first nucleotide sequence may encode domain III or a subdomain thereof, of murine CD154; and/or domain II, or subdomain thereof, of murine CD154; and/or domain I, or a subdomain thereof of murine CD154.

Methods of preparing CD154 chimeras, as well as the specific CD154 chimera ISF35 nucleic acid and protein sequences, will be apparent to one of skill in the art, and are described in U.S. Patent Application No. 2003/0220473 to Prussak, and/or U.S. Patent Application No. 2005/0048476, both of which are incorporated herein by reference in their entirety.

Further, in some embodiments of the invention, an extracellular domain of ISF35 can include at least one amino acid, or a sequence of amino acids, that may bind to anti-CD154 antibodies, and thereby neutralize or partially neutralize the immune-activating effect of the ligand. This amino acid or amino acid sequence in some embodiments is preferably the same or substantially similar to the regions in the tertiary structure of CD154 that bind to CD40, CD154's cognate receptor. It is believed that murine CD154 elicits a greater response in terms of anti-CD154 antibody production. As such, it is more sensitive than human CD154 to binding and neutralization by anti-CD154 antibodies, resulting in long-term problems with repeated administration of marine CD154 in humans.

In one embodiment, a ISF35 polynucleotide sequence or an analogous construct comprising a second nucleotide sequence of human CD154 that further encodes an extracellular subdomain to which anti-CD154 antibodies bind is provided. In this way, the polynucleotide sequence provided by the present invention encodes protein that is not immunogenic upon administration in humans.

The present specification also contemplates and includes an expression vector or any other genetic construct that comprises a polynucleotide sequence of the present invention capable of expressing ISF35 or an analogous construct in a target cell.

An expression vector useful contains polynucleotide sequence encoding ISF35 or an analogaus construct operatively linked to a suitable transcriptional or translations regulatory nucleotide sequence, such as one derived from a mammalian, microbial, viral, or insect gene. Such regulatory sequences include sequences having a regulatory role in gene expression, such as a transcriptional promoter or enhancer, an operator sequence to control transcription, a sequence encoding a ribosomal binding site within the messenger RNA, and appropriate sequences which control transcription, translation initiation, or transcription termination.

Particularly useful regulatory sequences include the promoter regions from various mammalian, viral, microbial, and insect senes The promoter region directs an initiation of transcription through and including the polynucleotide sequence encoding the ISF35 or an analogous construct of the present invention. Useful promoter regions include the promoter found in the Rous Sarcoma Virus (RSV) long terminal repeat (LTR), human cytomegalovirus (CMV) enhancer/promoter region, lac promoters, promoters isolated from adenovirus, and any other promoter known by one of ordinary skill in the art would understand to be useful for gene expression in eukaryotes, prokaryotes, viruses, or microbial cells. Other promoters that are particularly useful for expressing genes and proteins within eukaryotic cells/include mammalian cell promoter sequences and enhancer sequences such as those derived from polyoma virus, adenovirus, simian virus 40 (SV40), and the human cytomegalovirus. Particularly useful are the viral early and late promoters, which are typically found adjacent to the viral origin of replication in viruses such as the SV40. One of ordinary skill in the art will understand that the selection of a particular useful promoter depends on the exact cell lines and the other various, parameters of the genetic construct to be used to express a polynucleotide sequence within a particular cell line.

Certain genetic constructs contemplated by the present invention therefore include a polynucleotide sequence operatively linked to either a promoter sequence or a promoter and enhancer sequence and also operatively linked to a polyadenylation sequence that directs the termination and polyadenylation of messenger RNA. Preferably, the polynucleotide sequence is constructed using the CMV promoter and the bovine growth hormone polyadenylation sequence.

### Treatment of cancer by administering nucleic acids encoding ISF35 or an analogous construct as defined in the claims

ISF35 nucleic acids (and those of analogous constructs) can be administered to patients with cancer to treat or lessen the cancer progress. Methods for introducing polynucleotide sequences into specific cells in vivo, or within the subject's body are well known and include use of expression vectors and direct injection of various genetic constructs into the subject. In a typical application, an expression vector containing a polynucleotide sequence of the present invention is introduced into the circulation or at a localized site of the subject to allow the vector to specifically infect the desired cells. In other preferred embodiments the vector is injected directly into the tumor bed present in a subject that contains at least some of the cells into which the polynucleotide sequence of the present invention is to be introduced.

The present specification also contemplates and includes directly injecting into an animal or human subject a genetic construct that includes a polynucleotide sequence encoding ISF35 or an analogous construct, and may additionally include a promoter and a polyadenylation sequence. Examples of such useful methods have been described (Vile et al. Ann Oncol. 5:59-65, 1994). The genetic construct may also be directly injected into the muscle or other sites of an animal or human subject or directly into the tumor or tumor bed of the subject.

The present specification is also directed to methods of treating neoplasia, comprising inserting into a neoplastic cell a polynucleotide sequence of the present invention, so that the encoded 1SF35 or an analogous construct is expressed on the surface of the neoplastic cells. The present specification contemplates treating human neoplasis both in vivo and ex vivo.

In a preferred method of treating neoplasia, the method further comprises the steps of first obtaining the neoplastic cells from a subject, inserting therein a polynucleotide sequence of the present invention so that ISF35 or an analogous construct is expressed on the surface of the neoplastic cells, and re-administering the cells back into the subject. One of ordinary skill in the art will understand that numerous methods are applicable for re-administering the transformed neoplastic cells into the subject.

The ISF35 or an analogous construct can be used to treat many types of cancer. Exemplary cancers include but are not limited to breast cancer, lung cancer, colon cancer, prostate cancer, skin cancer, urinary bladder cancer, lyphoma, oral cavity cancer, pharynx cancer, leukemia, kidney cancer, pancreatic cancer, esophageal cancer, rectal cancer, bronchial cancer, stomach cancer, and the like.

Additional exemplary cancer types that can be treated using 1SF35 or on analogous construct include but are not limited to Acute Lymphoblastic Leukemia, Acute Myeloid Leukemia, Adrenocortical Carcinoma, AIDS-Related Cancers, ATIIS-Related Lymphoma, Anal Cancer, Astrocytoma, Basal Cell Carcinoma, Bile Duct Cancer, Bladder Cancer, Bone Cancer, Brain Stem Glioma, Brain Tumor, Breast Cancer, Bronchial Adenomas, Burkitt's Lymphoma, Carcinoid Tumor, Carcinoma, Central Nervous System Lymphoma, Cerebellar Astrocytoma, Cervical Cancer, Chronic Lymphocytic Leukemia, Chronic Myelogenous Leukemia, Chronic Myeloproliferative Disorders, Colon Cancer, Colorectal Cancer, Cutaneous T-Cell Lymphoma, Endometrial Cancer, Ependymoma, Esophageal Cancer, Extragonadal Germ Cell Tumor, Eye Cancer, Intraocular Metanoma, Eye Cancer, Retinoblastoma, Gallbladder Cancer, Gastrointestinal Carcinoid Tumor, Gastrointestinal Stromal Tumor (GIST), Germ Cell Tumor (Extracranial), Germ Cell Tumor (Extragonadal), Germ Cell Tumor (Ovarian), Gestational Trophoblastic Tumor, Glioma; Hairy Cell Leukemia, Head and Neck Cancer, Hepatocellular (Liver) Cancer, Hodgkin's Lymphoma, Hypopharyngeal Cancer, Hypothalamic and Visual Pathway Glioma, Intraocular Melanoma, Islet Cell Carcinoma (Endocrine Pancreas), Kaposi's Sarcoma, Kidney (Renal Cell) Cancer, Laryngeal Cancer, Leukemia (Acute Lymphoblastic), Leukemia (Acute Myeloid), Leukemia (Chronic Lymphocytic), Leukemia (Chronic Myelogenous), Lip and Oral CavityCancer, Liver Cancer, Lung Cancer (Non-Small Cell), Lung Cancer (Small Cell), Lymphoma, (Cutaneous T-Cell), Lymphoma (Non-Hodgkin's), Malignant Fibrous Histiocytoma of Bonc/Osteosarcoma, Medulloblastoma, Melanoma. Merkel Cell Carcinoma, Mesothelioma, Metastatic Squamous Neck Cancer with Occult Primary, Multiple Endocrine Neoplasia Syndrome, Multiple Myeloma/Plasma. Cell Neoplasm, Mycosis Fungoides, Myelodysplastic Syndromes, Myelodysplastic/Myeloproliferative Diseases, Myelogenous Leukemia, Myeloid Leukemia, Myeloproliferative Disorders, Nasal Cavity and Paranasal Sinus Cancer, Nasopharyngeal Cancer, Neuroblastoma, Oral Cancer, Oropharyngeal Cancer, Osteosarcoma/Malignant Fibrous Histiocytoma of Bone, Ovarian Cancer, Ovarian Epithelial Cancer, Ovarian Germ Cell Tumor, Ovarian Low Malignant Potential Tumor, Pancreatic Cancer, Parathyroid Cancer, Penile Cancer, Pheochromocytoma, Pineoblastoma and Supratentorial Primitive Neuroectodermal Tumors, Pituitary Tumor, Plasma Cell Neoplasm/Multiple Myeloma, Pleuropulmonary Blastoma, Prostate Cancer, Rectal Cancer, Retinoblastoma, Rhabdomyosarcoma, Salivary Gland Cancer, Sarcoma (Kaposi's), Sarcoma (uterine), Sezary Syndrome, Skin Cancer (non-Melanoma), Skin Cancer (Melanoma), Skin Carcinoma (Merkel Cell), Small Intestine Cancer, Soft Tissue Sarcoma, Squamous Cell Carcinoma, Stomach (Gastric) Cancer, T-Cell Lymphoma, Testicular Cancer, Thymoma, Thyroid Cancer, Trophoblastic Tumor, Gestational, Urethral Cancer, Uterine Cancer, Endometrial, Uterine Sarcoma, Vaginal Cancer, Visual Pathway and Hypothalamic Glioma, Vulvar Cancer, Waldenström's Macroglobulinemia, Wilms' Tumor, and the like.

A "chemotherapeutic agent" is a chemical compound useful in the treatment of cancer. A variety of chemical compounds, also described as "chemotherapeutic agents", function to induce DNA damage, all of which are intended to be of use in the combined treatment methods disclosed herein.

Accordingly, the ISF35 treatments can be combined with administration of chemotherapy agents. Exemplary chemotherapy treatments include but are not limited to adriamycin, doxorubicin, epirubicin, 5-fluorouracil, cytosine arabinoside ("Ara-C"), cyclophosphamide, thiotepa, busulfan, cytoxin, taxoids, e.g., paclitaxel (Taxol, Bristol-Myers Squibb Oncology, Princeton, NJ.), and doxetaxel (Taxotere, Rhone-Poulene Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins, melphalan, alemtuzimab, fludarabine, chlorambucil, rituxan, and the like.

Dosing of the chemotherapeutic agents can depend on the specific composition, the type of cancer to be treated, the condition of the patient, and other factors. In general, the dose can be administered by oral, intravenous, or other adminstration method as needed. A typical dosage can be from about 0.01 mg/m², 0.1 mg/m², 1 mg/m², 5 mg/m², 10 mg/m², 100 mg/m², 100 mg/m² to about 500 mg/m² depending on the type of agent, its purity, and other factors mentioned above.

For fludarabine, a typical dose for injection is about 25 mg/m² administered intravenously over a period of approximately 30 minutes daily for live consecutive days. Each 5 day course of treatment should commence about every 28 days. Dosage may be decreased or delayed based on evidence of hematologic or nonhematologic toxicity.

Alemtuzimab therapy can be initiated at a dose of about 3 mg administered as a 2 hour IV infusion daily. When the alemtuzimab 3 mg daily dose is tolerated (e.g., infusion-related toxicities are £ Grade 2), the daily dose can be escalated to about 10 mg and continued until tolerated. When the 10 mg dose is tolerated, the maintenance dose of alemtuzimab 30 mg can be initiated. The maintenance dose of alemtuzimab is typically about 30 mg/day administered three times per week on alternate days (i.e., Monday, Wednesday, and Friday) for up to about 12 weeks. In most patients, escalation to about 30 mg can be accomplished in about 3 - 7 days. Dose escalation to the recommended maintenance dose of about 30 mg administered three times per week is often required. Single doses of alemtuzimab greater than about 30 mg or cumulative weekly doses of greater that about 90 mg are typically not be administered since higher doses are associated with an increased incidence of pancytopenia.

Administration of the chemotherapeutic agent chlorambucil is typically by use of an oral dose. The usual oral dosage is 0.1 to 0.2 mg/kg body weight daily for 3 to 6 weeks as required. This usually amounts to 4 to 10 mg per day for the average patient. The entire daily dose may be given at one time.

The typical dose of the chemotherapeutic agent rituximab is about 375 mg/m² by IV infusion once a week for about 4 to about 8 doses. The range can be from about 5 mg/m² to about 500 mg/m².

Also described is that ISF35 makes cancer more sensitive to radiation treamtent and/or combinations of radiation and chemotherapy.

The invention is described in mort detail in the following examples. These examples are provided by way of illustration and are not intended to limit the invention in any way.

### EXAMPLES:

### Example I

### Adenovirus Synthesis

The chimeric ISF35 plasmid was digested with the restriction enymes Nruf and Sma I to release a DNA fragment containing the CMV promoter from pCDNA3, the ISF35 gene, and the polyadenylation signal from pCDNA3. Following gel purification of this fragment by separation of the digested DNA on a 1% agarose gel, the DNA fragment was ligated into the EcoRV site of the adenoviral shuttle vector MCS (SK) pXCX2. This plasmid is a modification of the plasmid pXCX2 such that the pBluescript polylinker sequence has been cloned into the El region, (J. R. Tozer, UCSD, unpublished data, September 1993). Following purification of chimeric ISF-MCS (SK) pXCX2 plasmid, 5 ug of this shuttle plasmid was cotransfected with 5 ug of JM17 plasmid into 293AC2 cells using the calcium phosphate Profection Kit from Promega according to the manufacturer's instructions. Following transfection, the cells were cultured for 5 days to allow for homologous recombination and viral synthesis. Total cells and supernatant were then harvested and freeze-thawed thrice to release cell. associated adenovirus.

Following the initial viral production, a clonal isolate of the virus obtained by plaque purification. Briefly, the freeze-thewed viral supernatant was cleared of debris by centrifugation at 1000 rpm in a tabletop centrifuge for 5 minutes. 293AC2 cells grown to confluency in 6 well tissue culture plates were then infected with serial dilutions of the viral supernatant for 1-2 hours. Following infection, the media was aspirated and cells overlayed with DMEM media containing 4% fetal calf serum and 0.65% agarose held at 56°C. Following 4.6 days incubation, isolated plaques were picked into 1 ml of media and subsequently used for viral amplification.

Large-scale adenovirus preparations were prepared by successively infecting increasing quantities of 293AC2. Purified adenovirus was then purified over cesium chloride step gradients. This method makes use of a cesium chloride gradient for concentrating virus particles via a step gradient, with the densities of 1.45 g/cm³ and 1.20 g/cm³. in which 293AC2 expanded virus samples are centrifuged for 2 hours in a SW40 rotor (Beckman, Brca, Calf.) at 25,000 rpm at 4°C. The virus band is isolated using a 27-gauge needle and syringe and desalted using a Sephadex G-25 DNA grade column (Pharmacia, Piscataway, N.J.). The virus is desalted against phosphate-buffered saline containing 10% glycerol and stored at -70°C. The final titer of the virus was determined by anion-exchange HPLC.

### Example 2

Expression and Function of a Chimeric Accessory Molecule Ligand Gene in CLL Cells and HeLa Cells HeLa cells were transiently transfected with ISF-pcDNA3 plasmid using lipofectamine 2000 (Gibco-BRL), a liposom-based transfection reagent allowing for efficient gene transfer into HeLa. Two days following transfection, cells were analyzed for cell surface expression of the ISF35 by flow cytometry. Briefly, the adherent cells are detached from the wells by aspirating the media and adding detaching solution (PBS containing 10 mM EDTA, pH B). This detaching solution is used in place of the of the more common trypsinization buffer to avoid nonspecific cleavage of ISF35 at trypsin sensitive sites, thus potentially leading to false negative assessment of expression. Once the cells detach from the plate, the cells are washed once in FACS staining buffer (composed of PBS containing 3% FCS and 0.05% sodium azide), resuspended in FACS buffer to approximately 10 sup.7 cells/ml, and 5X10⁵ (50 ul) cells are plated in 96 well u-bottom plastic microwell plates. PE-conjugated antibody specific for CD154 (antibody clone MR-1, Pharmingen, Inc.) is added for 30 minutes at 4°C. The cells are then washed twice with FACS buffer, resuspended in FACS buffer, and transferred to FACS tubes for data acquisition. To control for nonspecific antibody binding, all samples are stained with appropriate isotype control antibodies. Furthemore, dead cells and debris are excluded from analysis by addition of long/ml propidium iodide to all staining reactions. The cells are analyzed by flow cytometry for ISF35 expression using a FACSCalibur flow cctometer (Becton Dickinson). The results show that ISF35 is expressed as cell surface ligand that can be detected with CD154-specifc antibody, susgesting overall protein tertiary structure is maintained.

### Example 3

### Treatment of breast cancer patient with ISF35

A patient with breast cancer is identified. The patient is given a weekly dose of 3X10¹¹ ISF35 viral particles by intranodal injection. The patient is monitored twice per month. By use of this method, the breast cancer progression decreases.

### Example 4

### Treatment of a lung cancer patient with ISF35 in combination with a chemotherapeutic agent.

A patient with lung cancer is identified. The patient is given a one time dose of 2X10¹⁰ ISF35 viral particles by intranasal aerosol administration. After 3 days, the patient is given a Fludara injection at 25 mg/m² administered intravenously over 30 minutes a day for five days. The five day Fludara treatment is repeated every 28 days. The patient is monitored twice per month. By use of this method, the lung cancer progression decreases.

### Example 5

### Treatment of prostate cancer patient with ISF35 in combination with the chemotherapeutic agent rituximab.

A patient with prostate cancer is identified. The patient is given a once per month dose of 10X10¹¹ ISF35 viral particles by infection. Alter 1 week, the patient is given a rituxan injection at 375 mg/m² IV infusion once weekly for 8 doses. The patient ts monitored weekly. By use of this method, the prostate cancer progression decreases.

### Example 6

### Treatment of a CLL patient with ISF35 in combination with the chemotherapeutic agent chlorambucil.

A patient with CLL is identified. The patient is given a once per month injection of 3X10¹¹ ISF35 viral particles. After 1 week, the patient is given an oral dose of chlorambucil at 10 mg body weight daily for 5 weeks. The patient is monitored weekly. By use of this method, the CLL progression decreases.

## Claims

1. ISF35 for use in treating cancer, wherein an effective amount of ISF35 is to be administered to a patient prior to administration of an effective amount of a chemotherapeutic agent to said patient thereby treating cancer in said patient and making said cancer more sensitive to said chemotherapeutic agent than prior to the administration of ISF35 to said patient.

2. The ISF35 of claim 1, wherein the cancer is a refractory cancer.

3. The ISF35 of claim 1 or 2, wherein the ISF35 is to be administered at a dosage of from about 1x10⁵ to about 1x10¹² viral particles.

4. The ISF35 of claim 1, wherein the cancer is selected from the group consisting of breast cancer, lung cancer, colon cancer, prostate cancer, skin cancer, urinary bladder cancer, lymphoma, oral cavity cancer, pharynx cancer, leukemia, kidney cancer, pancreatic cancer, esophageal cancer, rectal cancer, bronchial cancer, and stomach cancer.

5. The ISF35 of any one of claims 1 to 4, wherein the chemotherapeutic agent is selected from the group consisting of fludarabine, alemtuzumab, chlorambucil, and rituximab.

6. The ISF35 of any one of claims 1 to 5, wherein radiation is also to be administered to the patient.

7. Chimeric CD154 polypeptide or nucleic acid encoding it for use in treating cancer in a patient, wherein an effective amount of a chimeric CD154 polypeptide or nucleic acid encoding it is to be administered to said patient prior to the administration of an effective amount of a chemotherapeutic agent to said patient thereby treating cancer in said patient and making said cancer more sensitive to said chemotherapeutic agent than prior to the administration of said chimeric CD154 polypeptide or nucleic acid encoding it to said patient.

## Patentansprüche

1. ISF35 zur Verwendung in der Behandlung von Krebs, wobei einem Patienten eine wirksame Menge von ISF35 vor der Verabreichung einer wirksamen Menge eines chemotherapeutischen Wirkstoffs an diesen Patienten zu verabreichen ist, wodurch der Krebs in dem Patienten behandelt wird und der Krebs sensitiver gegenüber dem chemotherapeutischen Mittel gemacht wird als vor der Verabreichung von ISF35 an den Patienten.

2. ISF35 nach Anspruch 1, wobei der Krebs ein refraktärer Krebs ist.

3. ISF35 nach Anspruch 1 oder 2, wobei das ISF35 in einer Dosierung von etwa 1x10⁵ bis etwa 1x10¹² viraler Partikel zu verabreichen ist.

4. ISF35 nach Anspruch 1, wobei der Krebs Brustkrebs, Lungenkrebs, Dickdarmkrebs, Prostatakrebs, Hautkrebs, Harnblasenkrebs, Lymphom, Mundhöhlenkrebs, Pharynxkrebs, Leukämie, Nierenkrebs, Pankreaskrebs, Speiseröhrenkrebs, Rektalkrebs, Bronchialkrebs oder Magenkrebs ist.

5. ISF35 nach einem der Ansprüche 1 bis 4, wobei der chemotherapeutische Wirkstoff Fludarabin, Alemtuzumab, Chlorambucil oder Rituximab ist.

6. ISF35 nach einem der Ansprüche 1 bis 5, wobei dem Patienten ferner Strahlung zu verabreichen ist.

7. Chimäres CD154-Polypeptid oder eine dieses kodierende Nukleinsäure zur Verwendung in der Behandlung von Krebs in einem Patienten, wobei dem Patienten eine wirksame Menge eines chimären CD154-Polypeptids oder einer dieses kodierenden Nukleinsäure vor der Verabreichung einer wirksamen Menge eines chemotherapeutischen Wirkstoffs an den Patienten zu verabreichen ist, wodurch der Krebs in dem Patienten behandelt wird und der Krebs sensitiver gegenüber dem chemotherapeutischen Mittel gemacht wird als vor der Verabreichung des chimären CD154-Polypeptids oder der dieses kodierenden Nukleinsäure an den Patienten.

## Revendications

1. ISF35 pour une utilisation dans le traitement du cancer, dans lequel une quantité efficace d'ISF35 est destinée à être administrée à un patient avant l'administration d'une quantité efficace d'un agent de chimiothérapie audit patient, traitant de cette manière un cancer chez ledit patient et rendant ledit cancer plus sensible audit agent de chimiothérapie qu'avant l'administration d'ISF35 audit patient.

2. ISF35 de la revendication 1, dans lequel le cancer est un cancer réfractaire.

3. ISF35 de la revendication 1 ou 2, dans lequel l'ISF35 est destiné à être administré à un dosage d'environ 1x10⁵ à environ 1x10¹² particules virales.

4. ISF35 de la revendication 1, dans lequel le cancer est choisi dans le groupe constitué d'un cancer du sein, un cancer du poumon, un cancer du colon, un cancer de la prostate, un cancer de la peau, un cancer de la vessie, un lymphome, un cancer de la cavité orale, un cancer du pharynx, une leucémie, un cancer du rein, un cancer du pancréas, un cancer de l'oesophage, un cancer rectal, un cancer des bronches et un cancer de l'estomac.

5. ISF35 selon l'une quelconque des revendications 1 à 4, dans lequel l'agent de chimiothérapie est choisi dans le groupe constitué de la fludarabine, l'alemtuzumab, le chlorambucile et le rituximab.

6. ISF35 selon l'une quelconque des revendications 1 à 5, dans lequel des radiations doivent également être administrées au patient.

7. Polypeptide CD154 chimérique ou acide nucléique le codant, pour une utilisation dans le traitement d'un cancer chez un patient, dans lequel une quantité efficace de polypeptide CD154 chimérique ou d'un acide nucléique le codant, est destinée à être administrée audit patient avant l'administration d'une quantité efficace d'un agent de chimiothérapie audit patient, traitant de cette manière un cancer chez ledit patient et rendant ledit cancer plus sensible audit agent de chimiothérapie qu'avant l'administration dudit polypeptide CD154 chimérique ou d'un acide nucléique le codant, audit patient.
